# EUROPEAN PATENT SPECIFICATION

(11) **EP 4 356 881 B1**
(45) Date of publication and mention of the grant of the patent: **26.08.2026**
(21) Application number: 23204501.3
(22) Date of filing: 18.10.2023
(51) Int. Cl.: A61F 9/007

(54) **A ROTARY VITRECTOMY PROBE**
ROTIERENDE VITREKTOMIESONDE
SONDE DE VITRECTOMIE ROTATIVE

(30) Priority: 19.10.2022 PL 44257922
(43) Date of publication of application: 24.04.2024
(73) Proprietor: Krasnicki, Pawel, 15-410 Bialystok (PL)
(72) Inventor: Krasnicki, Pawel, 15-410 Bialystok (PL)
(74) Representative: Wroblewski, Marcin Jan

(56) References cited:
- WO-A1-2022/148842
- US-A- 3 882 872
- US-A- 5 474 532
- US-A1- 2017 128 265

## Description

### FIELD OF THE INVENTION

The object of the invention is a rotary vitrectomy probe, a device which removes the vitreous body during ophthalmological procedures.

### STATE OF THE ART

To put it simply, a vitrectomy probe has a cannula which can be inserted into an eye, and which has a suction system and a moving element mounted in the cannula to cyclically cut off small portions of the vitreous body, sucked into the cannula.

Conventional vitrectomy probes usually comprise a hollow external cutting member, a hollow inner cutting member disposed coaxially and movably inside the hollow outer cutting member, and an opening extending radially across the external cutting member near its distal end. By means of a source of negative pressure, the vitreous body is sucked into the open opening, and the internal member is activated to close the open opening. While it is being closed, the cutting surfaces of both the internal and the external cutting element cooperate with each other to cut the vitreous body, following which the cut off vitreous body is sucked by the inner cutting element. The vitreous body is cut off piece by piece in order to avoid vitreomacular traction, which could lead to the strengthening of pathoses during surgery. As can be easily seen, the operation of this device is characterised by certain cyclicity: the opening opens gradually, sucking the vitreous body inside; afterwards, the inner cutting element falls down, cutting off the aspirated vitreous body and closing the port of the vitrectomy probe.

Therefore, the operation of the vitrectomy probe is pulsational. The consecutive distinguishable phases include the phase of increasing negative pressure with zero flow, the phase of rather rapid aspiration, and subsequently the cutting off phase, when the aspiration and flow once again drop to zero. The vitreous body is characterised by certain elasticity, which is why during vitrectomy it "jumps" away from the device after the cutting off phase, to subsequently be aspirated again with a high force. As can be seen, major changes in flow across the vitrectomy probe occur during the work cycle of this device, which causes the flow to be far from laminar. The device is highly "aggressive" and rather inefficient due to the generation of heavy turbulence around the port of the vitrectomy probe.

A guillotine style vitrectomy probe has an inner cutting element which moves with a reciprocating motion along its longitudinal axis. A rotary vitrectomy probe has an inner cutting element which moves with a rotary motion with respect to its longitudinal axis.

In both types of vitrectomy probes, the inner cutting elements are activated in various ways. For example, the internal cutting element may be moved from an open port position to a closed port position by pneumatic pressure exerted on a piston or membrane assembly, which overcomes a mechanical spring. Upon closing the inflow of pneumatic pressure, the spring retracts the internal cutting element from the closed port position to the open port position. As another example, the internal cutting element may be activated electromechanically between the open and closed port positions by means of a conventional rotary electric motor or a solenoid.

U.S. Patent no. 4.577.629 presents an example of a guillotine style pneumatic vitrectomy probe, activated with a piston/mechanical spring. Patent descriptions no. US4909249, US5019035 and US5047008A disclose guillotine style vitrectomy probes actuated pneumatically with a diaphragm/mechanical spring.

From prior art, there are also known rotary vitrectomy probes.

For example, these solutions are described in documents CA1233717A1, EP3442483B1, US5474532, US2017/0128265A1, WO202214884210A, US388872A.

Patent application CA1233717A1 describes an ophthalmic surgical instrument comprising rigid coaxial inner and outer tubular elements. Said outer tubular element has a closed distal end, near which a port for receiving the vitreous body being cut off is disposed on a side wall. Said inner tubular element has an open distal end. This end is bevelled at an angle relative to the longitudinal axis of the inner tubular element. It is characterised by three different types of conical configurations, and constitutes a sharpened cutting edge, which may open and close the port on the external tubular element.

Patent EP3442483B1 describes a vitrectomy probe with a body whose size and shape make it hand-graspable for the user. An outer cutting tube extending from the body has a port in its distal end for receiving the vitreous body being cut off. An inner cutting element rotatable around its longitudinal axis is disposed inside the outer cutting tube. The internal cutting element ends with a blade with one or more cutting surfaces, which rotate transversely to the external port for cutting the tissue, when the inner cutting element becomes rotated. The vitrectomy probe comprises a pneumatic vane motor disposed inside the body and coupled with the inner cutting element.

Patent US5474532 discloses a typical microsurgical instrument used to remove vitreous body, comprising an immovable outer cutting tube with vitrectome entrance and a pivotally mounted inner cutting tube. The inner tube at the distal end, at the level of the vitrectome port on the outer tube, takes the form of strips with cutting edges, arranged in parallel along its circumference, offset axially from each other by an equal angle. The effective aspiration area of the vitreous body is constant during the rotation of the inner cutting tube.

Application US2017/0128265A1 describes an eye treatment device with a tube-shaped outer housing. In the housing, there is a support on which, in turn, at least two rotating cutting elements are arranged circumferentially, preferably at equal intervals. The support may be tubular in shape. The outer casing has a hole through which the cutting element can contact the treated area of the eye. The cutting element is configured so that it can be rotated or deviated with respect to a predefined axis extending longitudinally or obliquely to it. It can be spiral-shaped.

The invention WO2022/14884210A provides a vitrectomy device comprising a housing and a cutting knife, the knife comprising an immovable outer tube connected to the housing, an inner tube located in the outer tube, the inner tube rotating about its longitudinal axis. The inner tube comprises multiple cutting edges at the distal end opposite the vitrectome port on the outer tube. The cutting edges of the inner tube and the cutting edge of the outer tube are configured to form a constant angle to each other due to the rotation of the inner tube. This causes one blade to cut at a time, while the others pull the aspirated vitreous body. Turbulence and uncontrolled pulling of the vitreous body occur.

The invention US3882872B is used to remove soft congenital cataracts, as well as hard calcified congenital cataracts, developmental cataracts, traumatic cataracts, including cataracts associated with dense fibrous retrobulbar membranes. In many of these cases, involvement of the vitreous body and complications are inevitable. In these situations, the current aspiration irrigation technique or ultrasound method would not be effective in treating dense fibrous retrobulbar cataracts, traumatic cataracts or calcified posterior polar congenital cataracts. Successful treatment of these cases also requires vitrectomy. This is easily done with the present invention and cannot be achieved with pre-existing techniques. The rotary knife according to the present invention generates more torque and allows the use of a variety of new tip configurations, thus providing greater surgical safety and versatility. Innovative tip configurations for shear and impact cutting were also disclosed.

At present, the companies manufacturing this device struggle with unwanted effects by increasing the number of cycles (cuts) per minute, which causes the device to be more "predictable" and less aggressive. This is because along with an increase in the working speed of the blade, the elastic vitreous body has less time to jump away from the vitrectomy probe when it is closed. In spite of the improved safety (small portions of the vitreous body being sucked in) and efficiency (a lower impact of the elasticity of the vitreous body), this solution is not perfect, since the original drawbacks have not been eliminated.

### THE ESSENCE OF THE INVENTION.

The invention is defined by the claims.

The essence of the solution according to the invention is a rotary vitrectomy probe comprising an immovable outer cutting tube with a vitrectomy port and an inner cutting tube mounted therein rotatably and coaxially with at least two cutting blades at the height of the vitrectome port, positioned at its periphery and displaced circumferentially with respect to each other by an equal angle, situated obliquely with respect to the longitudinal axis of the inner cutting tube, wherein the space remaining after the vitrectomy port is obscured by at least a portion of at least one cutting blade is the effective aspiration surface area of the vitreous body. The rotary vitrectomy is configured so that the effective aspiration surface area during rotation of the inner cutting tube relative to the outer cutting tube is constant. The cutting blades at the height of the vitrectome port are positioned obliquely with respect to the longitudinal axis of the inner cutting tube. The end parts of the outer cutting tube and the inner cutting tube at the height of the vitrectomy port preferably take a conical shape, tapering towards the distal end. The cutting blades on the conical part are situated with respect to the longitudinal axis of the inner cutting tube either in parallel or obliquely.

Preferably, the number of cutting blades is two, while the width of the port equals 1/2 of the circumference of the outer cutting tube.

Preferably, the number of cutting blades is three, while the width of the port equals 1/3 of the tube circumference of the outer cutting tube.

The oblique positioning of the cutting blades relative to the longitudinal axis of the inner cutting tube results in the cutting off of portions of the vitreous body not in an abrupt manner but in a gradual manner, further improving flow and smoothness of operation.

### EMBODIMENTS.

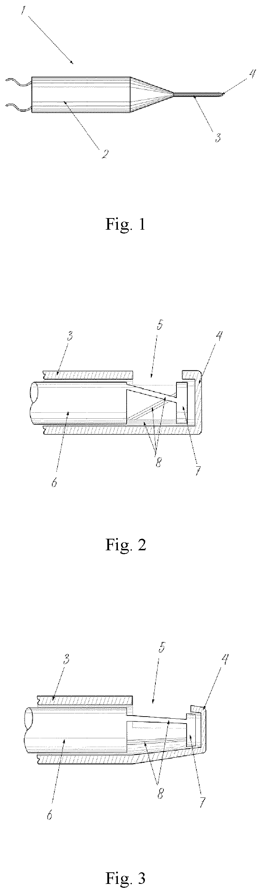

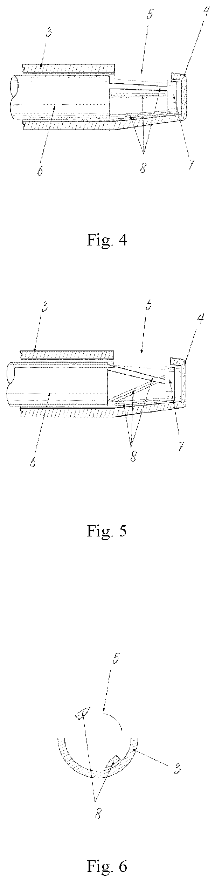

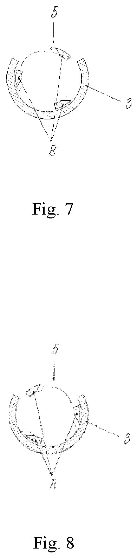

The object of the invention is shown in embodiments and in a drawing,
in which Fig. 1 shows the vitrectome in a longitudinal view; Fig. 2 - in a partial side view, the end of the outer tube of the vitrectome with three cutting blades on the inner tube, oblique to the longitudinal axis of the inner cutting tube; Fig. 3 - in a partial side view, the end of the outer tube of the vitrectome, the end parts of the outer tube and the inner tube of which, at the height of the vitrectome port, take a conical shape with two cutting blades on the inner tube, parallel to the longitudinal axis of the inner cutting tube; Fig. 4 - in a partial side view, the end of the outer tube of the vitrectome, the end parts of the outer tube and the inner tube of which, at the height of the vitrectome port, take a conical shape with three cutting blades on the inner tube, parallel to the longitudinal axis of the inner cutting tube; Fig. 5 - in a partial side view, the end of the outer tube of the vitrectome, the end parts of the outer tube and the inner tube of which, at the height of the vitrectome port, take a conical shape with three cutting blades on the inner tube, oblique to the longitudinal axis of the inner cutting tube; Fig. 6 - a transverse view of the outer tube at mid-height of the vitrectome port from Fig. 3, with two rotary cutting blades, offset circumferentially by an angle of 180° to each other; Fig. 7 - a transverse view of the outer tube at mid-height of the vitrectome port from Fig. 4, with three rotary cutting blades, offset circumferentially by an angle of 120° to each other; Fig. 8 - a transverse view of the outer tube at mid-height of the vitrectome port from Fig. 5, with three rotary cutting blades, offset circumferentially by an angle of 120° to each other.

### Embodiment 1.

The vitrectome 1 has a body 2, preferably cylindrical, with a size and shape that allow it to be grasped by the user's hand, in which a immovable cutting tube 3 is mounted. The distal end 4 of the tube 3 is blind, with a port 5, configured for aspiration of the vitreous body, cut in the lateral wall of the tube 3, near its distal end 4. Inside the cutting tube 3 there is a coaxial cutting tube 6 configured to rotate relative to the immovable outer tube 3. The inner cutting tube 6 at the distal end is blind with a tip 7, whereby at the height of the port 5 with a width of half the circumference of the tube 3 it takes the form of two cutting blades 8 situated obliquely with respect to the longitudinal axis of the inner cutting tube 6. The inner cutting tube 6 is driven by an actuator not shown in this figure.

### Embodiment 2.

The vitrectome 1 has a body 2, preferably cylindrical with a size and shape that allow it to be grasped by the user's hand, in which a immovable cutting tube 3 is mounted. The distal end 4 of the tube 3 is blind, with a port 5, configured for aspiration of the vitreous body, cut in the lateral wall of the tube 3, near its distal end 4. Inside the cutting tube 3 there is a coaxial cutting tube 6 configured to rotate relative to the immovable outer tube 3. The inner cutting tube 6 at the distal end is capped with a tip 7. The end parts of the outer tube 3 and the inner cutting tube 6 at the height of the vitrectome port 5 take a conical shape, while at the height of the port 5 with a width of half the circumference of the tube 3, it takes the form of two cutting blades 8 arranged in parallel to the longitudinal axis of the inner cutting tube 6. The inner cutting tube 6 is driven by an actuator not shown in this figure.

### Embodiment 3.

The vitrectome according to embodiment 2, wherein the cutting tube 6 at the height of the port 5 with a width of 1/3 of the circumference of the tube 3 takes the form of three cutting blades 8 located in parallel to the longitudinal axis of the inner cutting tube 6.

### Embodiment 4.

The vitrectome according to embodiment 2, wherein the cutting tube 6 at the height of the port 5 with a width of 1/3 of the circumference of the tube 3 takes the form of three cutting blades 8 located obliquely to the longitudinal axis of the inner cutting tube 6.

### ADVANTAGES OF THE SOLUTION.

The vitrectomy probe according to the invention is constructed so as to cut off portions of the vitreous body, at the same time aspirating it right behind the blade, which eliminates the effect of elasticity of the vitreous body. Moreover, in a vitrectomy probe operating in this manner, it is possible to reduce the negative pressure considerably (less aggressive operation, higher safety and predictability) as well as reduce the turbulence considerably, and make the flow of the vitreous body being removed similar to laminar, which improves the efficiency considerably.

Another advantage is the ability to use inclined blades, in which case portions of the vitreous body will not be cut off in a continuous manner, but gradually, which will additionally improve the flow and the work culture.

The conical shape of the vitrectomy probe next to its port provides undoubted advantages. Due to the above, apart from removing the vitreous body in a fluid and controlled manner, the vitrectomy probe in such an embodiment sucks it at a different angle. Apart from suction, the vitreous body also becomes detached from the surface of the retina. This may be very useful when detaching the vitreous body from the back during the procedure, as well as when eliminating vitreomacular traction. The fact that the size of the port of the vitrectomy probe is smaller near its base additionally enhances precision and safety when working near the surface of the retina.

## Claims

1. A rotary vitrectome probe comprising an immovable outer cutting tube (3) with a port (5) at its distal end and an inner cutting tube (6) coaxially and rotatably mounted in the outer cutting tube (3), with at least two cutting blades (8) at the height of the port (5) of the vitrectome probe, arranged on its periphery and offset circumferentially with respect to each other by an equal angle, wherein the space remaining after the vitrectome port (5) is obscured by at least a portion of at least one cutting blade (8) is the effective aspiration surface area of the vitreous body, and the rotating vitrectome probe (1) is configured so that the effective aspiration surface area during rotation of the inner cutting tube (6) relative to the outer cutting tube (3) is constant, **characterised in that**, the end parts of the outer cutting tube (3) and the inner cutting tube (6) starting at the height of the port (5) take a conical shape, tapering towards the distal end, and the cutting blades (8) on the conical part are positioned obliquely with respect to the longitudinal axis of the inner cutting tube.

2. The vitrectome probe according to claim 1, **characterised in that** the number of cutting blades (8) on the inner cutting tube (6) is two or three, depending on the configuration.

3. The vitrectome probe according to claim 1, **characterised in that** the number of cutting blades (8) is two when the width of the port (5) is equal to 1/2 of the circumference of the outer cutting tube (3).

4. The vitrectome probe according to claim 1, **characterised in that** the number of cutting blades (8) is three when the width of the port (5) is equal to 1/3 of the circumference of the outer cutting tube (3).

## Patentansprüche

1. Rotierende Vitrektomsonde umfassend ein unbewegliches äußeres Schneidrohr (3) mit einer Öffnung (5) an seinem distalen Ende sowie ein koaxial im äußeren Schneidrohr (3) angeordnetes und drehbar gelagertes inneres Schneidrohr (6), mit mindestens zwei Schneidklingen (8) auf der Höhe der Öffnung (5) der Vitrektomsonde, die an ihrem Umfang angeordnet und gegeneinander um den gleichen Umfangswinkel versetzt sind, wobei die nach der Verdeckung der Öffnung (5) der Vitrektomsonde durch mindestens einen Teil mindestens einer Schneidklinge (8) verbleibende Fläche die wirksame Aspirationsfläche für den Glaskörper bildet und die rotierende Vitrektomsonde (1) so ausgebildet ist, dass die wirksame Aspirationsfläche während der Rotation des inneren Schneidrohrs (6) relativ zum äußeren Schneidrohr (3) konstant bleibt, **dadurch gekennzeichnet, dass** die Endabschnitte des äußeren Schneidrohrs (3) und des inneren Schneidrohrs (6) beginnend auf der Höhe der Öffnung (5) konisch ausgebildet sind und sich zum distalen Ende hin verjüngen und dass die Schneidklingen (8) im konischen Abschnitt schräg zur Längsachse des inneren Schneidrohrsangeordnet sind.

2. Vitrektomsonde nach Anspruch 1, **dadurch gekennzeichnet, dass** die Anzahl der Schneidklingen (8) am inneren Schneidrohr (6) je nach Ausführung zwei oder drei beträgt.

3. Vitrektomsonde nach Anspruch 1, **dadurch gekennzeichnet, dass** die Anzahl der Schneidklingen (8) zwei beträgt, wenn die Breite der Öffnung (5) der Hälfte des Umfangs des äußeren Schneidrohrs (3) entspricht.

4. Vitrektomsonde nach Anspruch 1, **dadurch gekennzeichnet, dass** die Anzahl der Schneidklingen (8) drei beträgt, wenn die Breite der Öffnung (5) einem Drittel des Umfangs des äußeren Schneidrohrs (3) entspricht.

## Revendications

1. Sonde de vitrectome rotative comprenant un tube de coupe extérieur fixe (3) avec un orifice (5) à son extrémité distale et un tube de coupe intérieur (6) monté coaxialement et de manière rotative dans le tube de coupe extérieur (3), avec au moins deux lames de coupe (8) à la hauteur de l'orifice (5) de la sonde de vitrectome, disposées sur sa périphérie et décalées circonférentiellement les unes par rapport aux autres d'un angle égal, l'espace restant après occultation de l'orifice (5) du vitrectome par au moins une partie d'au moins une lame de coupe (8) constituant la surface effective d'aspiration du corps vitré, et la sonde de vitrectome rotative (1) étant configurée de sorte que la surface effective d'aspiration pendant la rotation du tube de coupe intérieur (6) par rapport au tube de coupe extérieur (3) soit constante, **caractérisé en ce que**, les parties terminales du tube de coupe extérieur (3) et du tube de coupe intérieur (6) commençant à la hauteur de l'orifice (5), présentent une forme conique se rétrécissant vers l'extrémité distale, et les lames de coupe (8) situées sur la partie conique sont positionnées obliquement par rapport à l'axe longitudinal du tube de coupe intérieur.

2. La sonde de vitrectome selon la revendication 1, **caractérisée en ce que** le nombre de lames de coupe (8) sur le tube de coupe intérieur (6) est de deux ou trois, selon la configuration.

3. La sonde de vitrectome selon la revendication 1, **caractérisée en ce que** le nombre de lames de coupe (8) est de deux lorsque la largeur de l'orifice (5) est égale à la moitié de la circonférence du tube de coupe extérieur (3).

4. La sonde de vitrectome selon la revendication 1, **caractérisée en ce que** le nombre de lames de coupe (8) est de trois lorsque la largeur de l'orifice (5) est égale à 1/3 de la circonférence du tube de coupe extérieur (3).
